# FASCICULE DE BREVET EUROPEEN

(11) **EP 3 297 565 B1**
(45) Date de publication et mention de la délivrance du brevet: **26.04.2023**
(21) Numéro de dépôt: 16733137.0
(22) Date de dépôt: 19.05.2016
(51) Int. Cl.: A61B 90/14, A61B 90/50, A61B 34/30

(54) **ROBOT D'ASSISTANCE NEUROCHIRURGICALE**
NEUROCHIRURGIEASSISTENZROBOTER
NEUROSURGICAL ASSISTANCE ROBOT

(30) Priorité: 21.05.2015 FR 1501055
(43) Date de publication de la demande: 28.03.2018
(73) Titulaire: Medtech SA, 34000 Montpellier (FR)
(72) Inventeur: NAHUM, Bertin, 34670 Baillargues (FR); BADANO, Fernand, 69100 Villeurbanne (FR); ROUSSEL, Eric, 34380 Mas De Londres (FR); BLONDEL, Lucien, 34000 Montpellier (FR)
(74) Mandataire: Taor, Simon Edward William
(86) Numéro de dépôt international: PCT/FR2016/051178
(87) Numéro de publication internationale: WO 2016/185140

(56) Documents cités:
- WO-A2-2009/013406
- WO-A2-2009/013406
- US-A- 5 997 176
- US-A- 6 033 415
- US-B1- 7 306 612

## Description

La présente invention entre dans le domaine médical et plus particulièrement dans le domaine de la neurochirurgie.

Elle concerne un robot d'assistance neurochirurgicale comprenant un bras support rigide permettant de positionner le robot par rapport à la tête d'un patient et de maintenir la tête du patient raisonnablement fixe au cours d'une opération neurochirurgicale.

On connaît au moins trois dispositifs médicaux permettant d'adapter la position d'un robot d'assistance neurochirurgicale en fonction de l'anatomie et de la position du patient, et d'assurer ainsi un maintien de la tête du patient au cours de l'opération neurochirurgicale.

Un premier dispositif connu est un robot d'assistance neurochirurgicale qui présente la particularité d'être immobilisé dans le bloc opératoire, notamment via un pilier scellé dans le sol. Ce dispositif présente plusieurs inconvénients principalement liés à son immobilisation au sol : il représente un encombrement important dans un bloc opératoire, il modifie la structure de ce dernier et entraîne de facto sa spécialisation à la neurochirurgie. De plus, son aspect rigide (fixation au sol) réduit ses possibilités d'adaptation au positionnement du patient.

Un deuxième dispositif médical du même ordre utilise les rails d'une table d'opération comme support de fixation d'une part pour une têtière supportant la tête du patient et d'autre part pour le robot d'assistance neurochirurgicale proprement dit. Un tel dispositif ne comprend pas de liaison rigide directe entre le robot et la tête du patient. De plus, plusieurs articulations mécaniques distinctes permettent la fixation de la têtière et du robot aux rails de la table d'opération, ce qui implique de multiples réglages pour adapter la fixation du dispositif chirurgical à la position du patient en vue de l'opération. Chaque articulation représente par ailleurs une perte potentielle de la rigidité du maintien de la tête du patient par rapport au robot,

Ce deuxième dispositif présente également l'inconvénient de conduire à un maintien de la têtière par rapport au robot qui dépend in fine de la rigidité de la table d'opération.

Un troisième dispositif médical, développé par le déposant, est un robot d'assistance neurochirurgicale qui conxprend un bras support rigide fixe. Au cours d'une opération, le bras support fixe est prévu pour maintenir de façon rigide la tête du patient via une têtière standard. Ce dispositif cceoprend donc une liaison rigide directe entre le robot et la tête du patient, mais il présente l'inconvénient d'être difficilement réglable à l'anatomie et surtout au positionnement du patient attaché sur la table d'opération. De plus, le fait que le bras support soit fixe et non amovible le rend plus encombrant, moins aisé à déplacer dans le contexte exigu d'une salle d'opération, et est enfin moins pratique à ranger en dehors de son utilisation. Certains dispositifs de maintient de la tête du patient sont décrits dans les documents suivants: WO 2009/013406 A2, US 6 033 415 A, US 5 997 176 A et US 7 306 612 B1.

La présente invention remédie aux insuffisances et problèmes précités, et vise è fournir un robot d'assistance neurochirurgicale ergonomique, c'est-à-dire en l'espèce facilement posltionnable par rapport à la position et à l'anatomie du patient tout en assurant, au cours d'une opération neurochirurgicale, un maintien suffisamment rigide et fiable de la tête du patient.

L'invention est exposée dans le jeu de revendications ci-joint.

Parmi les avantages procurés, le robot d'assistance neurochirurgicale de l'invention offre au surplus une amplitude, dans les réglages de positionnement, suffisante pour lui permettre de s'adapter à des configurations très variées de mise en place du robot.

A ces effets, le robot d'assistance neurochirurgicale de la présente invention, comportant classiquement un caisson mobile dont le châssis est muni d'un bras support rigide de liaison avec une têtière supportant la tête d'un patient allongé sur une table d'opération, est prévu tel que ledit bras support est mobile par rapport au châssis entre une position rétractée dans le volume du caisson et une pluralité de positions déployées, des moyens de verrouillage dudit bras en toute position étant prévus.

Les possibilités de rétractation et de réglage contribuent à la souplesse d'utilisation du robot de l'invention, aussi bien dans une perspective d'encombrement, et donc de gestion de l'espace dans une salle d'opération, que pour ce qui concerne la précision opératoire en accroissant l'adaptabilité positionnelle du robot relativement au patient.

Selon une configuration avantageuse, le châssis présente une structure rigide de référence comportant une plaque sous laquelle sont fixés des moyens de gestion de la mobilité du bras support et sur laquelle est fixé un second bras robotisé porteur d'outils.

L'un des aspects importants d'un robot chirurgical du type de l'invention réside dans la stabilité requise par la précision du geste chirurgical, stabilité qu'il convient en l'occurrence de faire rimer avec une variabilité/modularité dimensionnelle. L'objectif d'adaptation optimale à la position de la tête du patient ne doit pas être satisfait au détriment de la rigidité mécanique garante de cette stabilité. Une structure rigide de référence a donc été mise en place, munie d'un élément (la plaque) auquel sont directement solidarisés le bras robotisé chirurgical d'une part, et le bras support de la tête d'autre part, leur distribution par rapport à cette plaque renforçant encore la rigidité mécanique et la stabilité de l'ensemble.

En pratique, pour améliorer l'évaluation du positionnement relatif du robot et de son châssis par rapport à la table d'opération, le bras support peut comporter des moyens de repérage de sa position, permettant à un opérateur en phase préopératoire de prépositionner le bras robotisé supérieur par rapport à la tête du patient.

Plus précisément, selon une possibilité, le bras support peut comporter des graduations de repérage qui permettent de régler le bras support avec une grande précision, en combinaison avec des moyens de verrouillages adaptés.

Selon une configuration possible, qui répond bien à l'exigence de rigidité/stabilité posée auparavant, lesdits moyens de verrouillage sont reliés au châssis et peuvent comporter au moins un doigt indexeur sollicité par des moyens ressorts en direction d'au moins un évidement pratiqué dans le bras support rigide.

Dans ce cas, de préférence, le doigt indexeur peut comporter une extrémité libre conique et coopérer avec une succession d'orifices pratiqués selon la direction du déplacement dans une surface du bras support en regard dudit doigt. Cette conception permet une immobilisation fiable et robuste du bras support dans n'importe quelle position, même lorsque d'importantes contraintes opératoires sont exercées sur la tête du patient.

Selon un mode de réalisation, les moyens de verrouillage peuvent être activés/désactivés par une manette de commande mobile entre au moins deux positions correspondant respectivement à l'activation et à la désactivation des moyens de verrouillage.

De préférence, le bras support du robot d'assistance neurochirurgicale de l'invention est mobile selon un unique degré de liberté en translation par rapport au caisson. Dans ce cas, de préférence encore, la translation est d'axe horizontal.

Dans la perspective de la mise en œuvre d'un degré de liberté pour un réglage de position garantissant, pour chaque position stable du bras support de la têtière, une rigidité suffisante, le bras support selon l'invention comporte au moins un rail coulissant dans une glissière du châssis et coopérant avec au moins un patin de guidage solidaire fixé au châssis.

En pratique, le ou les patins de guidage sont disposés de préférence en sortie de la ou des glissières.

Toujours selon un mode de réalisation préférentiel, le bras support comporte en réalité deux rails latéraux coulissant dans deux glissières en regard fixées au châssis. Ce doublage fonctionnel joue un rôle dans la stabilisation du bras support par rapport au châssis, puisque les contraintes qui s'appliquent au bras durant ses déplacements sont supportées par deux rails et donc diminuées de moitié pour chaque rail,

Enfin, selon l'invention, l'extrémité libre du bras support peut être reliée à la têtière via une pièce radiotransparente fixée rigidement à l'extrémité libre dudit bras support et comportant une interface de liaison à la têtière gérant au moins deux degrés de liberté.

De manière générale, le système de fixation directe entre la tête du patient et le robot est réalisé avec un nombre réduit de pièces mécaniques, simples à fabriquer et à assembler, ce qui minimise avantageusement les risques de perte de stabilité/rigidité mécanique autant que les coûts de production. Un autre avantage de cette relative simplicité peut être vu dans une robustesse mécanique qui diminue sensiblement les nécessités d'entretien entre les utilisations.

Le système de liaison entre la tête du patient et le robot de l'invention permet au dispositif robotisé complet de présenter, du fait de la rétractabilité du bras support, des dimensions raisonnables à l'échelle d'une salle d'opération, l'encombrement correspondant à l'état rétracté le rendant de plus facile à ranger.

D'autres particularités et avantages de la présente invention apparaîtront dans la description détaillée d'un exemple de réalisation, non limitatif de l'invention, illustré dans les figures placées en annexe :
- La figure 1 est une représentation en vue perspective d'un robot d'assistance neurochirurgicale de l'invention dont le bras support est rétracté
- La figure 2 montre ledit robot, châssis apparent, avec le bras support partiellement déployé ;
- La figure 3 représente le châssis de ce robot, avec le bras support entièrement rétracté ;
- La figure 4 est une représentation dudit châssis avec le bras support entièrement déployé ;
- La figure 5 montre l'agencement du bras support et de la glissière dans laquelle il coulisse ;
- La figure 6 représente, en vue perspective partielle, les moyens de verrouillage prévus pour coopérer avec le bras support, en position non activée;
- La figure 7 montre lesdits moyens de verrouillage en position activée, bloquant rigidement le bras support ;

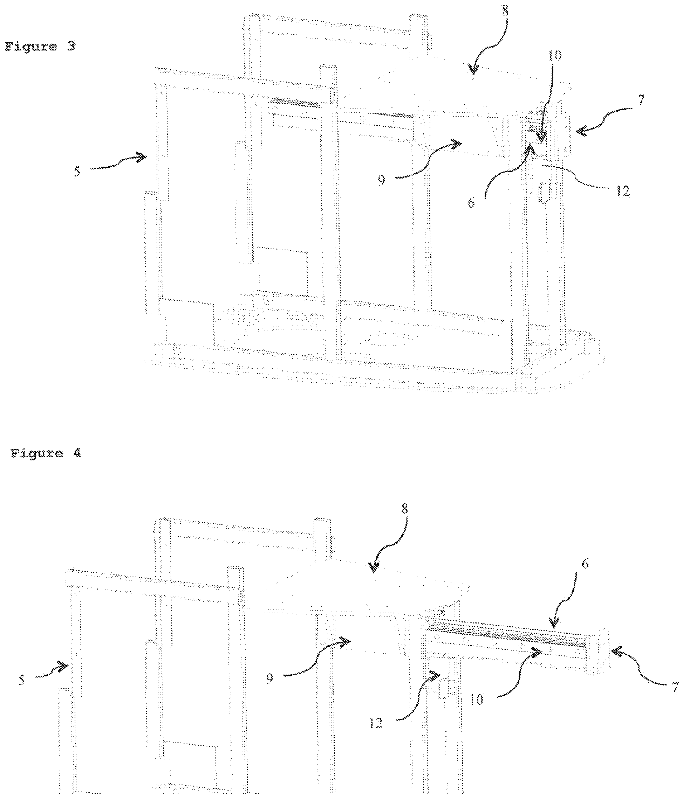

- La figure 3 illustre le fonctionnement du robot d'assistance neurochirurgicale de l'invention pour intervenir sur la boîte crânienne d'un patient allongé sur une table d'opération ; et

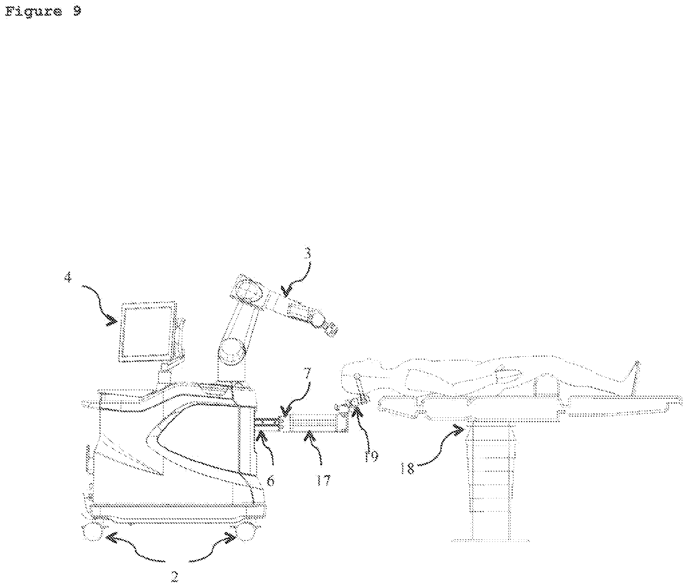

- La figure 9 représente l'interaction entre le robot et le patient en vue de côté.

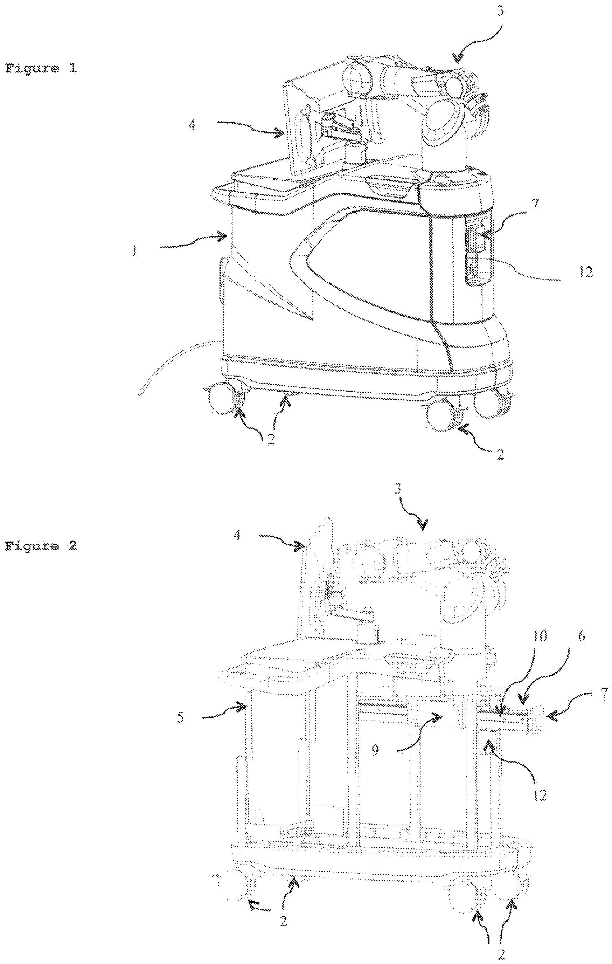

En référence à la figure 1, le robot d'assistance neurochirurgicale de l'invention comporte un caisson mobile (1) sur roulettes (2) surmonté d'un bras robotisé (3) dont l'extrémité libre est munie d'au moins un outil. Il présente par ailleurs un écran d'assistance (4) permettant à l'opérateur, en l'espèce le chirurgien, de visualiser l'opération en cours et plus précisément le travail de l'outil équipant le bras robotisé (3).

Le caisson mobile (1) habille un châssis (5) visible en figure 2, muni d'un bras support (6) rigide dont la fonction sera expliquée plus en détail dans la suite. Ledit bras support (6) est mobile par rapport au châssis (5) entre une position rétractée dans le volume du caisson (1), ou du châssis (5), et une pluralité de positions déployées. Il comporte une extrémité dotée d'une plaque de fixation (7) à des équipements notamment de maintien de la tête du patient.

En phase préopératoire, un opérateur adapte la position du robot d'assistance neurochirurgicale d'abord grossièrement en déplaçant le robot sur ses roulettes (2) puis plus précisément en déployant le bras support (6) en fonction du positionnement du patient, donc par rapport à l'emplacement de la table opératoire (18). Une fois la position optimale du bras support (6) établie, l'invention prévoit des moyens de verrouillage (12) permettant de maintenir ledit bras (6) de façon rigide dans pratiquement n'importe quelle position. Ainsi, la tête du patient est maintenue de façon rigide et surtout fiable au cours de l'opération. Le maintien de cette rigidité et de la précision du positionnement stable du caisson (1)/châssis (5) portant le bras robotisé (3) relativement au patient est évidemment tout à fait capital pour des applications à la chirurgie du cerveau.

La figure 1 montre le robot d'assistance neurochirurgicale avec le bras support (6) en position rétractée à l'intérieur du caisson (1). Avantageusement, dans cette configuration, le caisson mobile (1) est facilement manipulable et transportable dans un environnement encombré comme un bloc opératoire.

La figure 2 illustre plus particulièrement la façon dont le bras support (6) et le second bras robotisé (3) sont fixés au robot d'assistance neurochirurgicale, d'une manière qui garantit la stabilité nécessaire à un usage chirurgical lié au traitement du cerveau. Le châssis (5) comprend à cet effet une structure rigide de référence comportant notamment une plaque (8) plus particulièrement visible en figures 3 et 4, sous laquelle sont fixés des moyens de gestion (9) de la mobilité du bras support (6) (en l'occurrence en translation) et sur laquelle est fixé le second bras robotisé (3).

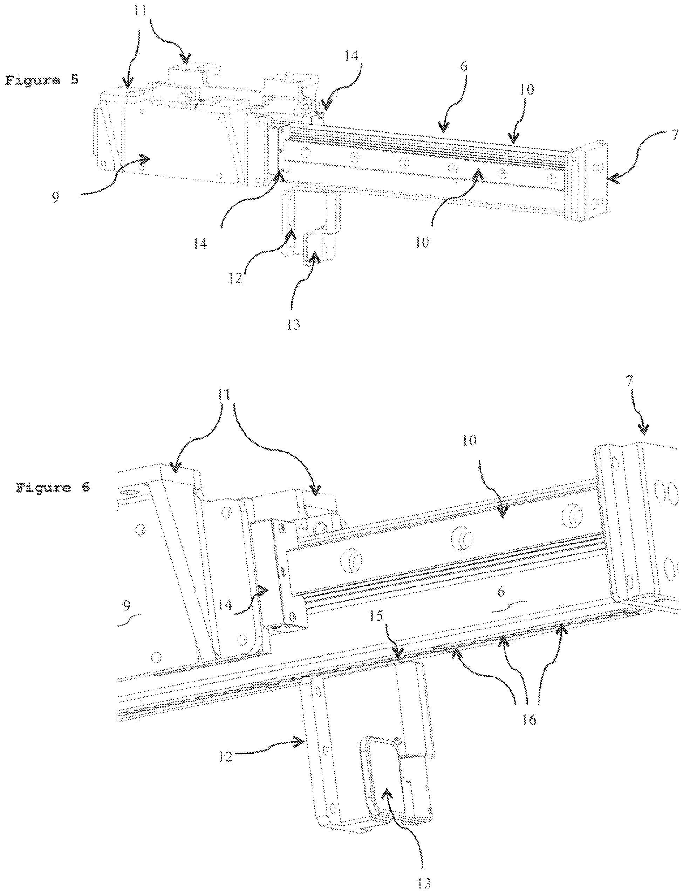

En pratique, le bras support (6) est mobile selon un unique degré de liberté en translation par rapport au châssis (5). Plus particulièrement, la translation du bras support (6) s'effectue selon un axe horizontal. A cet effet, le châssis (5) comprend un bloc de guidage (9) montré en figure 5, muni de deux glissières en vis-à-vis dans lesquelles coulissent deux rails latéraux (10). Ces derniers sont montés de part et d'autre sur les deux côtés d'allure verticale du bras support (6) de manière à correspondre aux emplacements des glissières dans le bloc (9). Cet agencement permet d'obtenir une translation horizontale étroitement guidée et donc très stable dudit bras support (6) entre deux positions extrêmes rétractée et déployée qui apparaissent respectivement aux figures 3 et 4.

Le bloc de guidage (9) est fixé sous la plaque (8) solidarisée au châssis (5)par exemple par boulonnage au niveau de consoles d'angle (11). Il comprend deux patins de guidages (14) disposés en sortie du bloc {9}, qui facilitent le glissement au cours du coulissement du bras support (6). Les patins de guidage (14) coopèrent de fait avec les rails (10) montés sur le bras support (6) et optimisent la précision de la translation du bras support (6).

Avantageusement, les patins de guidage (14) contribuent également à un maintien fiable de la position du bras support (6) en position déployée, et ce même lorsque d'importantes contraintes sont exercées sur la tête du patient, contraintes qui sont répercutées au bras (6) comme on le verra, dans la suite.

Pour assurer encore plus la stabilité et la rigidité de la liaison, en sus du guidage proprement dit, la forme du logement interne au bloc (9) correspond à la forme externe du bras (6), rails (10) inclus, ce qui améliore encore le positionnement de l'un dans l'autre à chaque instant au cours du déplacement et pour chaque position déployée.

Comme mentionné auparavant, le bras support (6) comporte des moyens de repérage (non représentés) qui sont par exemple constitués de graduations de repérage de la position dudit bras support (6). Cette caractéristique permet à l'opérateur notamment en phase préopératoire, de prépositionner avec une précision suffisante le robot d'assistance neurochirurgicale par rapport à la tête du patient.

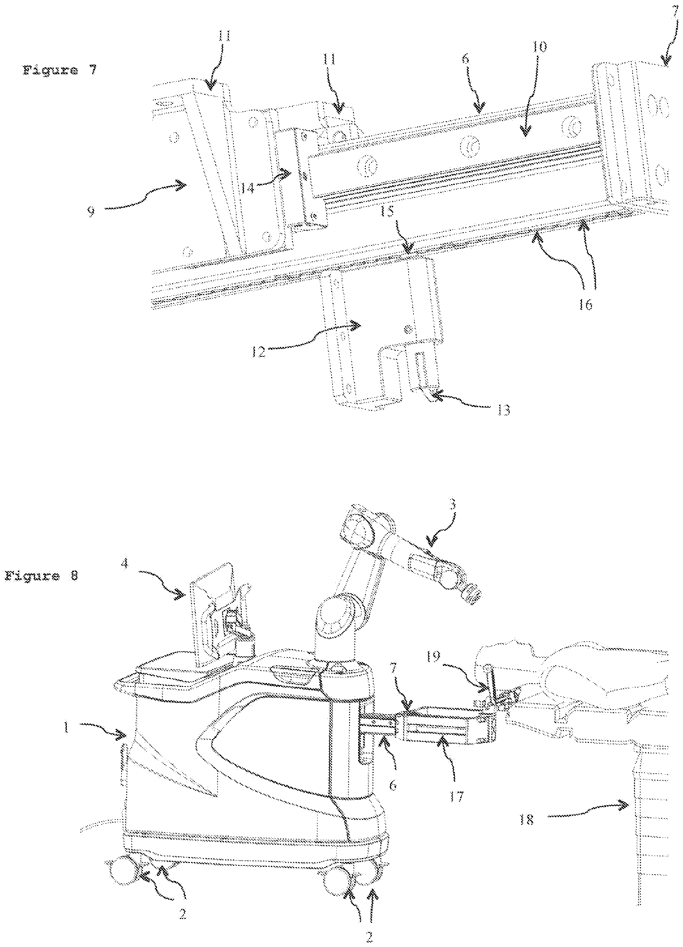

Les figures 6 et 7 illustrent plus particulièrement le fonctionnement des moyens de verrouillage (12) fixés au châssis (5), lesdits moyens (12) étant placés en sortie de la glissière (9) de façon à permettre un accès facile à la manette de commande (13).

Ces moyens de verrouillage (12) comportent au moins un doigt indexeur (15) dont l'extrémité conique, prévue à des fins de centrage dans des orifices (16) distribués à intervalle régulier dans la face inférieure du bras support (6), apparaît en figure 6. Ce doigt indexeur (15) est sollicité par des moyens ressorts en direction de ces orifices ou évidements (16) pratiqués dans le bras support (6). Les moyens ressorts peuvent par exemple être formés par un ressort de compression classique.

Plus précisément, l'extrémité libre conique du doigt (15), lorsqu'elle coopère avec les orifices (16) disposés selon la direction du déplacement, permet au bras support (6) de prendre un grand nombre de positions stables discrètes, avec une précision dimensionnelle qui dépend du diamètre des orifices. Dans l'application de l'invention, le doigt (15) s'ajuste avec un jeu minimal dans les orifices (16) de manière à empêcher tout déplacement incontrôlé, même faible, du bras support (6).

Les moyens de verrouillage (12) sont activés/désactivés par une manette de commande (13) mobile entre au moins deux positions, respectivement une position d'activation, qui apparaît en figure 7, et une position de désactivation montrée par exemple aux figures 5 et 6. L'utilisation d'une manette de commande (13) mobile telle que celle de l'invention permet un blocage rapide et efficace de la translation du bras support (6). L'extrémité conique du doigt (15), lorsqu'elle se centre dans un orifice (16), modifie légèrement le réglage dimensionnel initialement choisi, manuellement, par l'utilisateur dans sa phase de préréglage.

Comme illustré en figure 6, lorsque la manette de commande (13) est en position désactivée, le doigt indexeur (15) est rétracté dans son logement à l'encontre des moyens de ressorts, et l'opérateur peut alors librement ajuster la position du bras support (6) par translation.

Une fois la position optimale établie, l'opérateur déplace la manette de commande (13) mobile en position activée, débloquant les moyens de ressorts qui sollicitent et maintiennent l'extrémité conique du doigt indexeur (15) en buté dans l'orifice (16) lui faisant face, ou en tout état de cause dans l'orifice (16) la plus proche permettant un centrage la position et le déploiement du bras (6) étant alors verrouillés.

De tels moyens de verrouillage (12) permettent une immobilisation rapide, fiable et robuste du bras support (6) dans n'importe quelle position par blocage rigide du doigt (15) dans un orifice (16) de diamètre correspondant. La position de la tête du patient peut dès lors être maintenue de façon fiable, c'est-à-dire avec un degré d'immobilité suffisant de la structure qui la supporte, même lorsque d'importantes contraintes sont exercées sur la tête du patient.

Un mode de fixation possible de la tête du patient est d'ailleurs illustré aux figures 8 et 9 : l'extrémité libre du bras support (6) est en l'espèce reliée à un système (19) standard de fixation de la tête du patient allongé sur la table d'opération (18). Dans la configuration représentée, cette fixation est réalisée via une pièce radiotransparente (17) attachée à la plaque (7), qui prend la forme d'un cadre rigide. La radiotransparence de ladite pièce (17) permet, en phase peropératoire, de réaliser et d'obtenir des clichés à l'aide d'imageurs utilisant des rayons X en éliminant toutes radio-nuisances dans l'environnement proche de la tête du patient.

Le cadre radiotransparent (17) doit absolument être fixé de façon rigide à l'extrémité libre du bras support (6), puisque c'est sur lui qu'est solidarisé le système (19) standard de fixation de la tête du patient, par l'intermédiaire d'une interface de liaison gérant au moins deux degrés de liberté. La mobilité relative, quand elle est nécessaire, est donc prise en charge à cet endroit de la chaîne mécanique.

Les degrés de liberté de la pièce d'interface, par exemple attachée à une têtière standard (19) comme représenté sur les figures 8 et 9, permettent d'adapter de façon encore plus précise le positionnement du robot d'assistance neurochirurgicale par rapport à la tête du patient en cours d'opération. La gestion mécanique des degrés de liberté y est prévue au plus près de la tête du patient. Ainsi lorsque d'importantes contraintes sont exercées sur la tête du patient, ce qui arrive au cours d'opérations impliquant des phases de travail préliminaires sur les parties osseuses de la boîte crânienne, le couple ou les contraintes exercé(es) sur chaque liaison gérant un degré de liberté est ou sont réduit(es) au maximum.

Dans l'exemple représenté, l'interface de liaison entre le cadre radiotransparent (17) et la têtière standard (19) présente cinq degrés de liberté.

Il existe des alternatives à l'utilisation d'une têtière standard (19) pour maintenir la tête du patient, qui sont parfaitement utilisables dans le cadre de la présente invention, par exemple les dispositifs basés sur un cadre stéréotaxique à deux degrés de liberté.

Cette possibilité n'est pas représentée : de manière générale, la configuration décrite au moyen des figures n'est pas exhaustive de l'invention, qui englobe les variantes de forme et de structure dont la portée est limitée par les revendications, notamment mais non exclusivement pour ce qui concerne le réglage dimensionnel du robot de l'invention en position déployée.

## Revendications

1. Un appareil robotisé pour des opérations neurochirurgicales, l'appareil robotisé comportant :
un bras robotisé (3) dont l'extrémité libre est conçue pour tenir au moins un outil chirurgical ;
un châssis (5) supportant le bras robotisé et habillé d'un caisson mobile (1) ;
un bras support rigide (6) couplé de manière mobile avec le châssis (5) et extensible à partir d'une première position rétractée dans le châssis à une pluralité de positions déployées où au minimum une partie du bras support rigide s'étend à l'exterieur du caisson mobile ; et
un mécanisme de verrouillage (12) fixé au châssis (5) et coopérant avec le bras support rigide (6) pour le verrouillage du bras support rigide dans l'une des quelconque pluralités de position déployées,
**caractérisé en ce que** l'extrémité libre du bras (6) est reliée à une têtière (19) pour le maintien d'une tête d'un patient, via une pièce radiotransparente (17) fixée rigidement à l'extrémité libre dudit bras support (6) et comportant une interface de liaison à la têtière (19) gérant au moins deux degrés de liberté.

2. L'appareil robotisé selon la revendication 1, **caractérisé en ce que** le châssis (5) comporte une structure rigide de référence comportant une plaque (8) sous laquelle sont fixés des moyens (9) de gestion de la mobilité du bras support (6), **caractérisé en ce que** le bras robotisé (3) est fixé sur la plaque (8).

3. L'appareil robotisé selon l'une des revendications précédentes, **caractérisé en ce que** le bras support (6) comporte des moyens de repérage de sa position.

4. L'appareil robotisé selon la revendication 3, **caractérisé en ce que** le bras support (6) comporte des graduations de repérage.

5. L'appareil robotisé selon l'une quelconque des revendications précédentes, **caractérisé en ce que** le mécanisme de verrouillage (12) comporte au moins un doigt indexeur (15) sollicité par des moyens ressorts en direction d'au moins un évidement (16) pratiqué dans le bras support (6) rigide.

6. L'appareil robotisé selon la revendication 5, **caractérisé en ce que** le doigt indexeur (15) comporte une extrémité libre conique et coopère avec une succession d'orifices (16) pratiqués selon la direction du déplacement dans une surface du bras support (6) en regard dudit doigt (15).

7. L'appareil robotisé selon l'une quelconque des revendications précédentes, **caractérisé en ce que** le mécanisme de verrouillage (12) est activé/désactivé par une manette de commande (13) mobile entre au moine deux positions correspondant respectivement à l'activation et à la désactivation du mécanisme de verrouillage (12).

8. L'appareil robotisé selon la revendication précédente, **caractérisé en ce que** le bras support (6) est mobile selon un unique degré de liberté en translation par rapport au caisson (1).

9. L'appareil robotisé selon la revendication 8, **caractérisé en ce que** la translation est d'axe horizontal.

10. L'appareil robotisé selon l'une quelconque des revendications précédentes, **caractérisé en ce que** le bras support (6) comporte au moins un rail coulissant (10) dans une glissière du châssis (5) et coopérant avec au moins un patin de guidage (14) solidarisé au châssis (5).

11. L'appareil robotisé selon la revendication précédente, **caractérisé en ce que** le ou les patins de guidage(14) sont disposés en sortie de la ou des glissières.

12. L'appareil robotisé selon l'une des revendications 10 et 11, **caractérisé en ce que** le bras support (6) comporte deux rails latéraux (10) coulissant dans deux glissières en regard fixées au châssis (5).

## Patentansprüche

1. Robotervorrichtung für neurochirurgische Operationen, wobei die Robotervorrichtung Folgendes beinhaltet:
einen Roboterarm (3), dessen freies Ende ausgelegt ist, um mindestens ein chirurgisches Werkzeug zu halten;
ein Gehäuse (5), das den Roboterarm stützt und mit einem Wechselkasten (1) ausgestattet ist;
einen starren Stützarm (6), der auf bewegliche Weise mit dem Gehäuse (5) gekoppelt und aus einer ersten eingezogenen Position in dem Gehäuse zu einer Vielzahl von ausgefahrenen Positionen erweiterbar ist, bei denen sich mindestens ein Teil des starren Stützarms außerhalb des Wechselkastens erstreckt; und
einen Verriegelungsmechanismus (12), der an dem Gehäuse (5) befestigt ist und mit dem starren Stützarm (6) zusammenwirkt, um den starren Stützarm in einer aus der Vielzahl von ausgefahrenen Positionen zu verriegeln,
**dadurch gekennzeichnet, dass** das freie Ende des Arms (6) mit einem Kopfstück (19) für den Halt eines Kopfes eines Patienten über ein strahlendurchlässiges Teil (17) verbunden ist, das starr an dem freien Ende des Stützarms (6) befestigt ist und eine Verbindungsschnittstelle mit dem Kopfstück (19) beinhaltet, die mindestens zwei Freiheitsgrade steuert.

2. Robotervorrichtung nach Anspruch 1, **dadurch gekennzeichnet, dass** das Gehäuse (5) eine starre Referenzstruktur beinhaltet, die eine Platte (8) beinhaltet, unter der Mittel (9) zur Steuerung der Beweglichkeit des Stützarms (6) befestigt sind, **dadurch gekennzeichnet, dass** der Roboterarm (3) auf der Platte (8) befestigt ist.

3. Robotervorrichtung nach einem der vorhergehenden Ansprüche, **dadurch gekennzeichnet, dass** der Stützarm (6) Mittel zur Kennzeichnung seiner Position beinhaltet.

4. Robotervorrichtung nach Anspruch 3, **dadurch gekennzeichnet, dass** der Stützarm (6) Kennzeichnungsabstufungen beinhaltet.

5. Robotervorrichtung nach einem der vorhergehenden Ansprüche, **dadurch gekennzeichnet, dass** der Verriegelungsmechanismus (12) mindestens einen Indexierfinger (15) beinhaltet, der mit Federmitteln in Richtung von mindestens einer Aussparung (16) beaufschlagt ist, die in dem Stützarm (6) umgesetzt ist.

6. Robotervorrichtung nach Anspruch 5, **dadurch gekennzeichnet, dass** der Indexierfinger (15) ein konisches freies Ende beinhaltet und mit einer Abfolge von Öffnungen (16) zusammenwirkt, die in der Verschiebungsrichtung in einer Oberfläche des Stützarms (6) in Bezug auf den Finger (15) umgesetzt sind.

7. Robotervorrichtung nach einem der vorhergehenden Ansprüche, **dadurch gekennzeichnet, dass** der Verriegelungsmechanismus (12) durch einen Bedienungsgriff (13), der zwischen mindestens zwei Positionen beweglich ist, die jeweils der Aktivierung und der Deaktivierung des Verriegelungsmechanismus (12) entsprechen, aktiviert/deaktiviert wird.

8. Robotervorrichtung nach dem vorhergehenden Anspruch, **dadurch gekennzeichnet, dass** der Stützarm (6) gemäß einem einzigen Freiheitsgrad in Verschiebung in Bezug auf den Kasten (1) beweglich ist.

9. Robotervorrichtung nach Anspruch 8, **dadurch gekennzeichnet, dass** die Verschiebung auf der horizontalen Achse ist.

10. Robotervorrichtung nach einem der vorhergehenden Ansprüche, **dadurch gekennzeichnet, dass** der Stützarm (6) mindestens eine Gleitschiene (10) in einer Führung des Gehäuses (5) beinhaltet und mit mindestens einem Gleitschuh (14) zusammenwirkt, der einstückig mit dem Gehäuse (5) verbunden ist.

11. Robotervorrichtung nach dem vorhergehenden Anspruch, **dadurch gekennzeichnet, dass** der oder die Gleitschuhe (14) am Ausgang der einen oder mehreren Führungen angeordnet sind.

12. Robotervorrichtung nach einem der Ansprüche 10 und 11, **dadurch gekennzeichnet, dass** der Stützarm (6) zwei seitliche Schienen (10) beinhaltet, die in zwei Führungen gleiten, die an dem Gehäuse (5) befestigt sind.

## Claims

1. A robotic apparatus for neurosurgical operations, said robotic apparatus comprising:
a robotic arm (3) the free end of which is adapted to hold at least one surgical tool;
a chassis (5) supporting the robotic arm and covered with a movable box (1);
a rigid support arm (6) movably coupled with the chassis (5) and extendable from a first retracted position within the chassis to a plurality of deployed positions where at least a portion of the rigid support arm extends outside the movable box; and
a locking mechanism (12) fastened to the chassis (5) and cooperating with the rigid support arm (6) for locking the rigid support arm in any one of the plurality of extended positions,
**characterised in that** the free end of the arm (6) is connected to a headrest (19) for maintaining a patient's head, via a radiotransparent member (17) fastened rigidly to the free end of said support arm (6) and comprising a connecting interface with the headrest (19) providing at least two degrees of freedom.

2. Robotic apparatus according to claim 1, **characterised in that** the chassis (5) has a rigid reference structure including a plate (8) under which are fastened means (9) for managing the mobility of the support arm (6), **characterised in that** the robotic arm (3) is fastened to the plate (8).

3. Robotic apparatus according to any one of the preceding claims, **characterised in that** the support arm (6) comprises means for marking its position.

4. Robotic apparatus according to claim 3, **characterised in that** the support arm (6) comprises reference graduations.

5. Robotic apparatus according to any one of the preceding claims, **characterised in that** the locking mechanism (12) comprises at least one indexing finger (15) biased by spring means towards at least one recess (16) made in the rigid support arm (6).

6. Robotic apparatus according to claim 5, **characterised in that** the indexing finger (15) comprises a conical free end and cooperates with a succession of openings (16) made along the direction of movement in a surface of the support arm (6) opposite to said finger (15).

7. Robotic apparatus according to any one of the preceding claims, **characterised in that** the locking mechanism (12) is activated/deactivated by a control handle (13) movable between at least two positions corresponding respectively to the activation and deactivation of the locking mechanism (12).

8. Robotic apparatus according to the preceding claim, **characterised in that** the support arm (6) is movable with one degree of freedom in translation relative to the box (1).

9. Robotic apparatus according to claim 8, **characterised in that** the translation is in the horizontal axis.

10. Robotic apparatus according to the preceding claim, **characterised in that** the support arm (6) comprises at least one rail (10) sliding in a slide of the chassis (5) and cooperating with at least one guide shoe (14) rigidly connected to the chassis (5).

11. Robotic apparatus according to the preceding claim, **characterised in that** the guide shoe or shoes (14) is or are arranged at the exit of the slide or slides.

12. Robotic apparatus according to any one of claims 10 and 11, **characterised in that** the support arm (6) comprises two lateral rails (10) sliding in two opposite slides fastened to the chassis (5).
